# FASCICULE DE BREVET EUROPEEN

(11) **EP 1 450 883 B1**
(45) Date de publication et mention de la délivrance du brevet: **30.05.2007**
(21) Numéro de dépôt: 02799790.7
(22) Date de dépôt: 06.12.2002
(51) Int. Cl.: A61M 5/168

(54) **DISPOSITIF DE CONTROLE DE DEBIT, A USAGE MEDICAL**
DURCHFLUSSKONTROLLVORRICHTUNG, INSBESONDERE ZUM MEDIZINISCHEN GEBRAUCH
FLOW RATE CONTROL DEVICE FOR MEDICAL USE

(30) Priorité: 06.12.2001 FR 0115794
(43) Date de publication de la demande: 01.09.2004
(73) Titulaire: Sobem, 01100 Arbent (FR); Mermet, Bernard, Matafelon-Granges 01580 (FR)
(72) Inventeur: MERMET, Bernard, F-01580 Matafelon-Granges (FR)
(74) Mandataire: Maureau, Philippe
(86) Numéro de dépôt international: PCT/FR2002/004217
(87) Numéro de publication internationale: WO 2003/047660

(56) Documents cités:
- WO-A-02/15965
- US-A- 4 335 729
- US-A- 5 113 904
- US-A- 6 156 025
- PATENT ABSTRACTS OF JAPAN vol. 015, no. 403 (C-0875), 15 octobre 1991 (1991-10-15) & JP 03 165778 A (NIPPON MEDICAL SUPPLY CORP), 17 juillet 1991 (1991-07-17)

## Description

La présente invention concerne un dispositif de réglage de débit, notamment à usage médical.

Un tel dispositif est couramment utilisé notamment dans les systèmes de perfusion pour régler le débit du liquide perfusé.

Le terme contrôle de débit doit être pris au sens large et vise également des distributeurs de débit à une ou plusieurs voies avec des fonctions ouverture et fermeture. ,

Un dispositif connu comprend deux pièces reliées l'une à l'autre de manière pivotante, dont une délimite un conduit destiné à être raccordé à une source de fluide, notamment une poche de liquide à perfuser, et dont l'autre délimite un conduit destiné à être raccordé à un tuyau d'acheminement du liquide.

Suivant une première forme d'exécution, l'une desdites pièces comprend un fond dans lequel est aménagée, coaxialement à l'axe de pivotement d'une pièce par rapport à l'autre, une rainure circulaire s'étendant sur légèrement moins d'un tour et ayant une profondeur variable sur sa longueur ; le conduit que comprend cette pièce débouche dans cette rainure. L'autre pièce est solidaire en rotation d'une rondelle d'étanchéité en matériau résilient, présentant un orifice venant en communication d'une part avec le conduit de cette autre pièce et d'autre part avec ladite rainure circulaire.

Le pivotement de cette autre pièce par rapport à la première pièce citée permet le déplacement de l'orifice de la rondelle d'étanchéité le long de la rainure et donc, du fait de la profondeur différente de cette rainure en différents emplacements de celle-ci, de faire varier la section du conduit d'écoulement du fluide selon la position angulaire d'une pièce par rapport à l'autre.

L'obtention de l'étanchéité requise implique un serrage de la rondelle entre les deux pièces du dispositif, de sorte que des contraintes relativement importantes sont générées sur cette rondelle.

Ces contraintes interdisent une stérilisation du dispositif à la vapeur. En effet, l'élévation de température conduirait à un ramollissement de la matière constituant lesdites pièces et, accessoirement ladite rondelle, et, compte tenu des contraintes précitées, à un risque de fluage de ces matières, altérant l'étanchéité et le fonctionnement du dispositif, voire même risquant de rendre ce dernier impropre à l'usage.

Pour cette raison, la stérilisation d'un dispositif de ce type est actuellement réalisée soit au moyen d'un rayonnement de particules soit au moyen d'un gaz permettant une stérilisation à faible température, tel que de l'oxyde d'éthylène.

Ces modes de stérilisation ont pour inconvénient essentiel d'impliquer de grandes précautions d'emploi et donc d'être relativement onéreux à mettre en oeuvre. L'emploi d'un gaz de stérilisation à faible température a également pour inconvénient de provoquer une diffusion de produits chimiques dans l'environnement.

Le document WO 02/15965 constitue un état de la technique selon l'art 54(3). Il décrit un dispositif d'administration d'insuline à débit constant réglable. Le dispositif est destiné à être fixé sur la peau d'un utilisateur et comporte une canule destinée à pénétrer dans la peau de ce dernier. Le dispositif comporte égaiement deux pièces déplaçables l'une par rapport à l'autre et des moyens de réglage de débit actionnés par le déplacement de la première pièce par rapport à la seconde pièce.

L'invention vise à remédier à ces inconvénients, en fournissant un dispositif de réglage à même d'être stérilisé au moyen de vapeur.

L'invention a également pour objectif de fournir un dispositif de réglage présentant une parfaite étanchéité et ayant une structure optimisée du point de vue de sa fabrication.

Le dispositif qu'elle concerne comprend, de manière connue en soi, deux pièces reliées l'une à l'autre avec possibilité de déplacement d'une pièce par rapport à l'autre, un organe d'étanchéité placé entre ces deux pièces, et des moyens de réglage de débit actionnés par le déplacement d'une pièce par rapport à l'autre l'une des pièces délimitant un conduit destiné à être raccordé à une source de fluide et l'autre pièce délimitant un conduit destiné à être raccordé à un tuyau d'acheminement dudit fluide.

Selon l'invention, le dispositif comprend des moyens de positionnement permettant de placer les deux pièces dans deux positions différentes, à savoir une première position, dite de "stérilisation", dans laquelle ces deux pièces n'exercent pas, ou exercent peu, de contraintes sur cet organe d'étanchéité, et une deuxième position, dite de "fonctionnement", dans laquelle ces deux pièces serrent entre elles ledit organe d'étanchéité de manière à obtenir l'étanchéité requise.

Les deux pièces du dispositif sont placées en position de "stérilisation" au moment de la stérilisation du dispositif, pour annuler toutes les contraintes que subissent ces pièces lorsqu'elles exercent un serrage de l'organe d'étanchéité afin d'obtenir l'étanchéité requise. Il devient alors possible de stériliser ce dispositif à la vapeur, sans que l'échauffement résultant de ce type de stérilisation génère un risque de fluage de la matière constituant ces pièces et l'organe d'étanchéité. Une telle stérilisation est donc rendue possible sans altération de l'étanchéité et du fonctionnement du dispositif.

De préférence, lesdits moyens de positionnement comprennent :
- au moins une rainure aménagée dans l'une desdites pièces ;
- au moins un bossage solidaire de l'autre pièce, coulissant dans cette rainure pour permettre le déplacement des pièces l'une par rapport à l'autre ; et
- au moins un dégagement communiquant avec la rainure, dans lequel peut être engagé le bossage pour permettre le déplacement de la pièce comprenant la rainure par rapport à la pièce comprenant le bossage, de ladite position de fonctionnement à ladite position de stérilisation, et inversement.

Ce déplacement est ainsi rendu possible de manière simple à fabriquer et pratique à utiliser.

Avantageusement, chaque rainure et/ou chaque bossage présente des zones inclinées formant des rampes, ces rampes favorisant le passage des pièces de la position de stérilisation à la position de fonctionnement nonobstant la force de rappel élastique de l'organe d'étanchéité.

Selon une forme de réalisation préférée de l'invention lorsque le dispositif comprend au moins une rainure et au moins un bossage tel que précités, la rainure présente au moins une entrée dans laquelle un bossage peut être engagé dans une position déterminée d'une pièce par rapport à l'autre, et l'une des pièces comprend au moins un cran de verrouillage tandis que l'autre pièce comprend au moins un cran de verrouillage correspondant, ces crans étant conformés de telle sorte que le ou les crans d'une pièce peuvent s'encliqueter au-delà du ou des crans de l'autre pièce lors du premier déplacement d'une pièce par rapport à l'autre et être ensuite verrouillés au-delà de ce ou ces derniers de manière à interdire tout retour des pièces vers la position dans laquelle chaque bossage se trouve en regard de chaque entrée.

L'assemblage du dispositif peut ainsi être réalisé de manière particulièrement simple et facile à réaliser.

De préférence, l'organe d'étanchéité présente une partie d'étanchéité ayant une section transversale en forme de "U" et lesdites pièces présentent des formes telles que, lorsqu'elles sont assemblées, elles enserrent cette partie d'étanchéité dans toutes les directions.

Une parfaite étanchéité est ainsi obtenue.

Selon une forme de réalisation préférée de l'invention, lesdites pièces sont reliées de manière pivotante l'une par rapport à l'autre, l'une desdites pièces comprend une paroi dans laquelle est aménagée, coaxialement à l'axe de pivotement d'une pièce par rapport à l'autre, une rainure circulaire s'étendant sur légèrement moins d'un tour et ayant une profondeur variable sur sa longueur, et le conduit que comprend cette pièce débouche dans cette rainure ; l'autre pièce est solidaire en rotation de l'organe d'étanchéité, qui présente un orifice venant en communication d'une part avec le conduit de cette autre pièce et d'autre part avec ladite rainure circulaire ; le pivotement de cette autre pièce par rapport à la première pièce citée permet le déplacement de l'orifice de l'organe d'étanchéité le long de la rainure et donc, du fait de la profondeur différente de cette rainure en différents emplacements de celle-ci, de faire varier la section du conduit d'écoulement du fluide selon la position angulaire d'une pièce par rapport à l'autre.

L'une des pièces du dispositif peut comprendre au moins une fenêtre débouchant sur l'extérieur et l'autre pièce du dispositif peut être solidaire en rotation d'une bague comportant des informations relatives au débit réglé au moyen du dispositif, ces informations venant en regard de ladite fenêtre dans les différentes positions angulaires de la pièce solidaire de la bague par rapport à la pièce comportant la fenêtre.

Selon une autre forme d'exécution, les moyens d'étanchéité sont constitués par deux surfaces coniques complémentaires prenant appui l'une contre l'autre et appartenant respectivement aux pièces (2, 3).

Pour sa bonne compréhension, l'invention est à nouveau décrite ci-dessous en référence au dessin schématique annexé représentant, à titre d'exemple non limitatif, deux formes de réalisation du dispositif qu'elle concerne.
La figure 1 est une vue en perspective des pièces d'un dispositif de réglage de débit, avant assemblage ;
la figure 2 en est une vue en perspective de ces pièces, sous un autre angle ;
la figure 3 est une vue en perspective, à échelle agrandie, d'une pièce qu'il comprend, avec arrachement partiel ;
la figure 4 est une vue en perspective d'une autre pièce qu'il comprend, également à échelle agrandie ;
la figure 5 est une vue des pièces qu'il comprend, en coupe passant par l'axe de ces pièces, avant assemblage, et
la figure 6 est une vue similaire à la figure 5, à échelle agrandie, après assemblage desdites pièces.
les figures 7 et 8 représentent un robinet à trois voies vu en coupe transversale et en coupe longitudinale, respectivement.

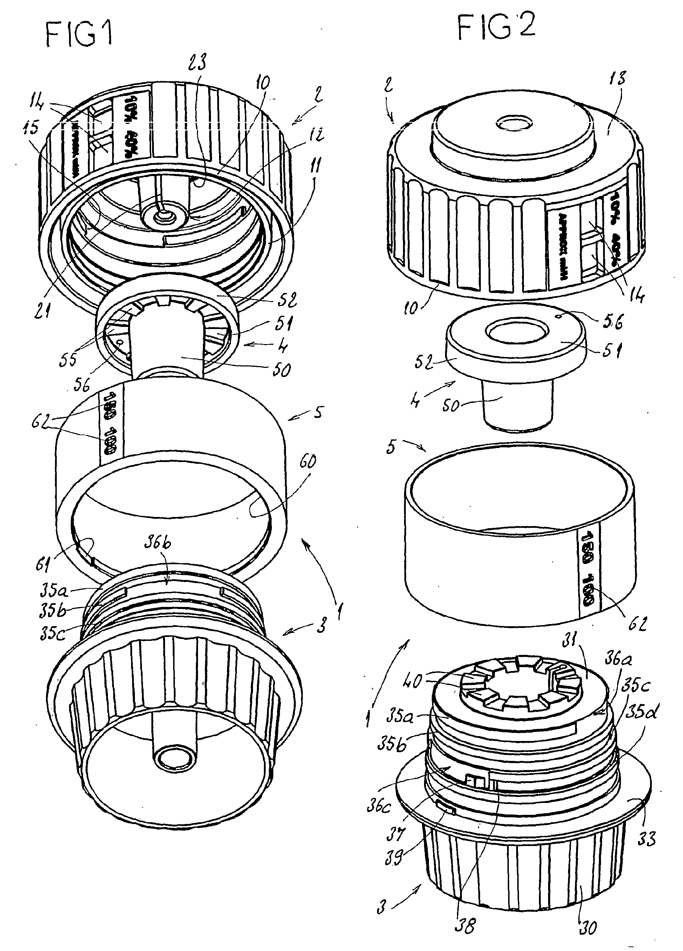

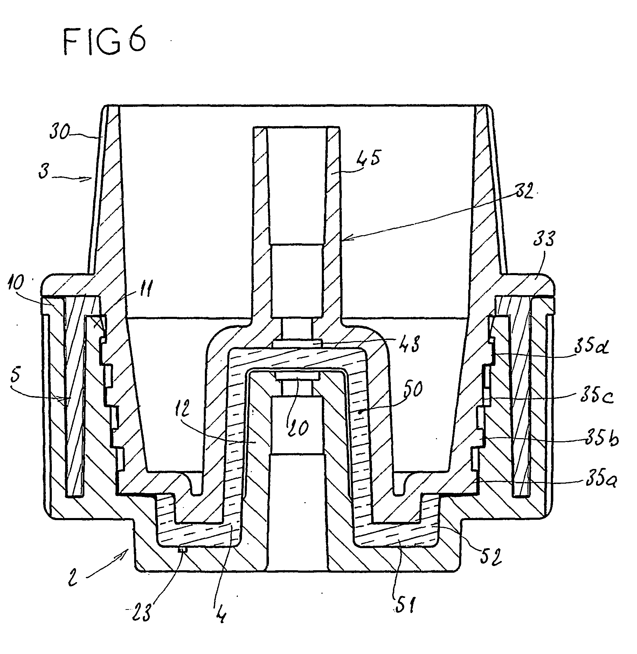

Les figures 1 à 6 représentent un dispositif 1 de réglage de débit, à usage médical.

Ce dispositif 1 comprend une première pièce 2, une deuxième pièce 3 destinée à être reliée de manière pivotante à la pièce 2, un organe d'étanchéité 4 et une bague 5.

La pièce 2 comprend une paroi périphérique extérieure 10, une paroi périphérique intérieure 11, un plot central creux 12, un fond 13, deux fenêtres 14 aménagées dans la paroi 10, trois bossages 15 faisant saillie de la face interne de la paroi 11 radialement vers l'intérieur, et un cran de verrouillage 16 faisant également saillie de cette même face interne, radialement vers l'intérieur.

La paroi 10 présente des reliefs antidérapants sur sa face extérieure.

Comme le montrent les figures, la paroi 11 est aménagée à distance de la paroi 10, de sorte que ces parois 10, 11 délimitent entre elles un espace annulaire dans lequel peut être engagée et peut pivoter la bague 5.

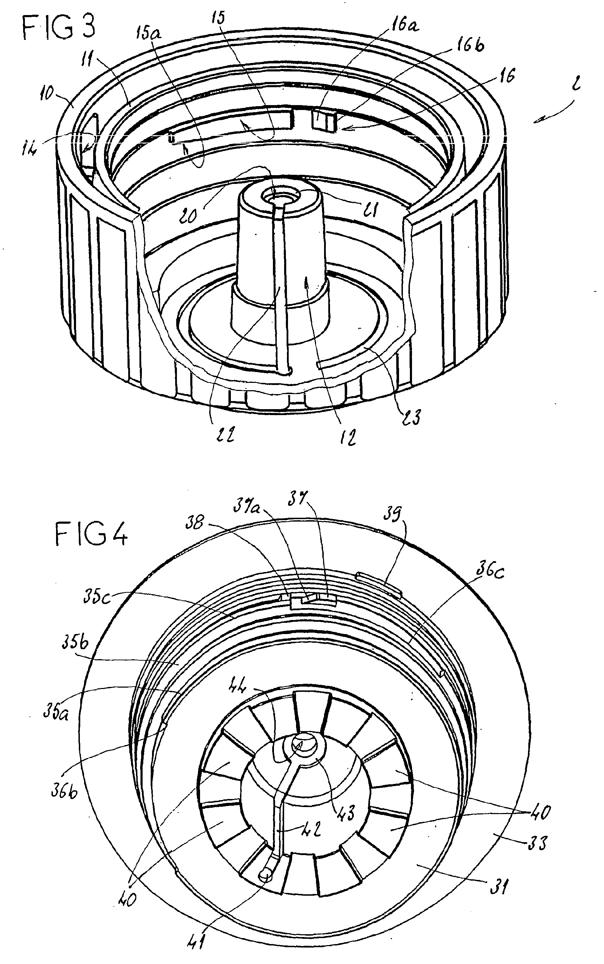

Le plot 12 est aménagé coaxialement à la pièce 2. Il apparaît plus particulièrement sur !a figure 3 qu'il présente un trou axial 20 communiquant, par une rainure circulaire 21 et une rainure longitudinale 22, avec une rainure circulaire 23 aménagée dans le fond 13, coaxialement au plot 12. Il apparaît sur la figure 3 que cette rainure 23 s'étend sur légèrement moins d'un tour et qu'elle a une profondeur variable sur sa longueur.

Le trou 20 communique avec l'espace délimité intérieurement par le plot 12, qui débouche lui-même sur l'extérieur de la pièce 2.

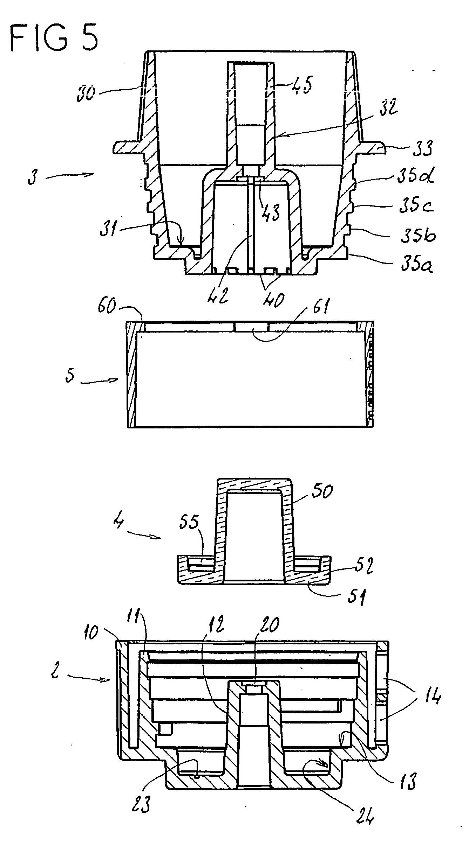

La figure 5 montre que le fond 13 forme un évidement circulaire 24 autour du plot 12.

Les bossages 15 sont disposés à 120 degrés les uns des autres et sont situés sur trois hauteurs différentes de la paroi 11. Ils présentent des zones entaillées au niveau de leurs bords tournés vers le fond 13 et au niveau de l'une de leurs extrémités, ces zones formant ainsi des parois inclinées 15a.

Le cran 16 est aménagé près du plot 15 situé le plus en hauteur, du côté de celui-ci opposé à l'extrémité présentant la paroi 15a. Ce cran 16 est conformé pour réaliser un encliquetage, c'est-à-dire présente une paroi inclinée 16a et une paroi 16b orientée de manière radiale.

La pièce 3 comprend une paroi périphérique 30, un fond 31 et un plot central creux 32.

La paroi 30 présente une collerette 33 faisant saillie de sa face extérieure, sensiblement à mi-hauteur d'elle, cette collerette 33 étant destinée à venir en appui contre le bord supérieur de la paroi 10 lorsque les pièces 2 et 3 sont assemblées l'une à l'autre.

Cette collerette 33 délimite une partie "extérieure" de la paroi 30, c'est-à-dire située à l'extérieur du dispositif 1 après assemblage de ce dernier, et une partie "intérieure" de cette paroi 30, c'est-à-dire destinée à être engagée dans la pièce 2.

La partie extérieure présente des reliefs antidérapants sur sa face extérieure.

La partie intérieure comprend des nervures 35a, 35b, 35c, 35d faisant saillie radialement vers l'extérieur, qui définissent entre elles trois gorges superposées. La nervure 35a la plus proche du fond 31 s'interrompt sur une distance angulaire supérieure à la longueur angulaire d'un bossage 15, délimitant ainsi une zone 36a d'entrée de l'un de ces bossages 15 dans la gorge la plus proche du fond 31. La nervure 35b s'interrompt de la même manière sur une distance angulaire supérieure à la longueur angulaire d'un bossage 15, mais en un emplacement décalé de 120 degrés par rapport à la zone d'entrée 36a, délimitant ainsi une zone 36b d'entrée de l'un des bossages 15 dans la gorge intermédiaire. De la même façon, la nervure 35c s'interrompt sur une distance angulaire supérieure à la longueur angulaire d'un bossage 15, mais en un emplacement décalé de 120 degrés par rapport à la zone d'entrée 36b, délimitant ainsi une zone 36c d'entrée de l'un des bossages 15 dans la gorge la plus éloignée du fond 31.

La paroi 30 comprend en outre un cran d'encliquetage 37 et une butée de positionnement 38, prolongeant vers le haut l'extrémité voisine de la nervure 35c. La paroi inclinée 37a du cran 37 est tournée vers la butée 38.

En dessous de la collerette 33, la pièce 3 comprend un ergot 39 faisant saillie radialement vers l'extérieur.

Le fond 31 comprend une couronne de créneaux radiaux 40 faisant saillie axialement de sa face extérieure.

En outre, comme le montre plus particulièrement la figure 4, le fond 31 présente un trou 41 qui le traverse, relié, par une rainure longitudinale 42 et une rainure circulaire 43, à un orifice 44 aménagé dans un épaulement que comprend le plot 32. La distance du trou 41 par rapport à l'axe de révolution de la pièce 3 correspond au rayon de la rainure circulaire 23, de sorte que ce trou 41 se trouve en regard de cette rainure 23 et peut parcourir cette dernière lorsque la pièce 3 est pivotée par rapport à la pièce 2.

Le plot 32 est aménagé coaxialement à la pièce 3. Au-delà de la paroi formant l'épaulement précité, il présente une tubulure de raccordement 45, par exemple de type Luer, faisant saillie du côté opposé au fond 31.

L'organe d'étanchéité 4 est réalisé en une matière résiliente telle qu'un silicone. Il présente un plot central creux 50, un fond 51 et un bord périphérique relevé 52.

Le plot 50 est dimensionné extérieurement pour s'engager étroitement dans la partie inférieure du plot 32 et est dimensionné intérieurement pour être engagé sur le plot 12 avec possibilité de pivotement par rapport à celui-ci.

Le fond 51 présente des créneaux radiaux 55 propres à s'engager étroitement dans les espaces délimités par les créneaux 40, et inversement, de manière à assurer une liaison en rotation entre cet organe 4 et la pièce 3. Les dimensions de ce fond 51, ainsi que celles du bord périphérique 52 sont telles que ce fond 51 et ce bord 52 peuvent être engagés étroitement dans l'évidement circulaire 24, mais avec possibilité de pivotement par rapport à la pièce 2.

Ce fond 51 comprend un trou 56 venant en coïncidence du trou 41 lorsque l'organe 4 est assemblé à la pièce 3.

La bague 5 présente un rebord 60 faisant saillie radialement vers l'intérieur et une encoche 61 aménagée dans ce rebord 60. Ce rebord 60 permet l'engagement de la bague 5 sur la paroi 30 jusqu'à engagement de l'ergot 39 dans l'encoche 61, ce qui assure le calage en rotation de cette bague 5 par rapport à la pièce 3.

La bague 5 comporte en outre une série d'informations 62 relatives au débit pouvant être réglé par le dispositif 1, imprimées sur sa face extérieure, qui viennent en regard des fenêtres 14 dans les différentes positions angulaires de la pièce 3 par rapport à la pièce 2. Ces informations 62 renseignent ainsi l'utilisateur sur le débit réglé.

L'assemblage du dispositif 1 est réalisé en engageant l'organe 4 sur la pièce 3, en engageant la bague 5 dans l'espace circulaire délimité par les parois 10 et 11 puis en engageant l'ensemble pièce 3 / organe 4 dans la pièce 2, avec les bossages 15 situés en regard des zones 36a à 36c. Au niveau de la zone 36c, la butée 38 dirige le cran 16 dans l'espace délimité entre cette butée 38 et la paroi inclinée 37a du cran 37. Lorsque la pièce 3 est pivotée par rapport à la pièce 2 dans le sens horaire sur l'exemple représenté sur les figures, les parois inclinées 16a et 37a des crans 16 et 37 glissent l'une sur l'autre ce qui, par déformation élastique de la pièce 3, amène le cran 16 à passer au-delà du cran 37 et à être verrouillé derrière celui-ci. L'assemblage des pièces 3 et 2 est ainsi devenu irréversible.

Dans cet état d'assemblage, l'orifice 20, les rainures 21, 22 et 23, les trous 56 et 41, les rainures 42 et 43 et l'orifice 44 délimitent un conduit traversant le dispositif 1 de part en part. Le fond de la pièce 2 peut être relié à une source de liquide à perfuser, telle qu'une poche souple, et la tubulure 45 peut être reliée à un tuyau d'acheminement du liquide vers un patient.

Le pivotement de la pièce 3 par rapport à la pièce 2 permet de déplacer le trou 56 le long de la rainure 23 et donc de faire varier, selon la position angulaire de ce trou 56 par rapport à cette gorge 23, la section transversale dudit conduit traversant le dispositif 1, et donc le débit obtenu.

Les nervures 35a à 35d et les bossages 15 sont aménagés de telle sorte que, lorsque les bossages 15 sont engagés dans les gorges délimitées par ces nervures 35a à 35d, les pièces 2 et 3 serrent l'organe 4 entre elles. Ces nervures 35a à 35d et ces bossages 15 permettent ainsi d'obtenir l'étanchéité requise entre ces pièces 2 et 3.

Lorsque les bossages 15 se trouvent dans les zones 36a à 36c, la pièce 3 peut jouer axialement par rapport à la pièce 2, entre la position de fonctionnement du dispositif 1, dans laquelle les pièces 2 et 3 serrent l'organe 4 entre elles, et une position de stérilisation, dans laquelle la contrainte exercée sur cet organe 4 par les pièces 2 et 3 est relâchée.

Les parois inclinées 15a des bossages 15 permettent de réengager facilement les bossages 15 dans les gorges délimitées par les nervures 35a à 35d et de recomprimer facilement l'organe 4 lorsqu'il s'agit de repasser de la position de stérilisation à la position de fonctionnement.

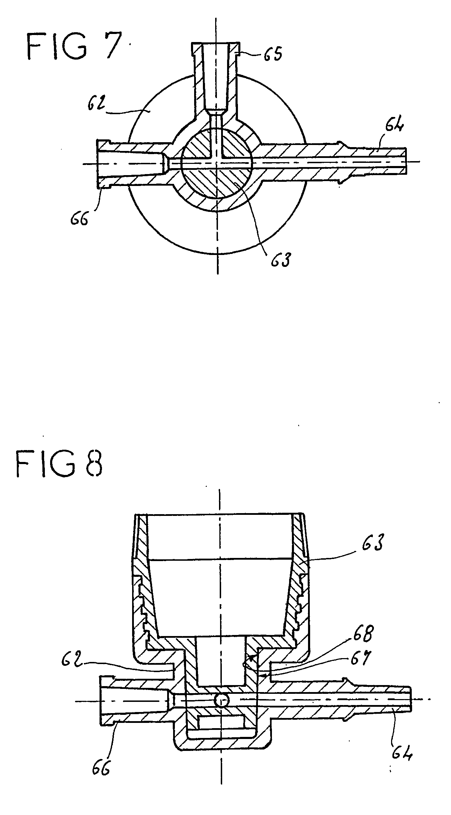

Les figures 7 et 8 représentent un robinet à trois voies, comprenant un corps 62 dans lequel est monté un boisseau 63. Trois tubes (64, 65, 66) débouchent au centre du corps, et servent au montage de trois tubulures non représentées au dessin. L'étanchéité entre le corps 62 et le boisseau 63 est réalisée par des surfaces coniques 67, 68 appartenant respectivement au corps et au boisseau, en appui l'une contre l'autre. La structure permettant le passage du boisseau de la position stérilisation à la position de fonctionnement étant la même que précédemment n'est pas décrite ici.

Ainsi qu'il apparaît de ce qui précède, l'invention apporte une amélioration déterminante à la technique antérieure, en fournissant un dispositif de réglage de débit à usage médical à même d'être stérilisé au moyen de vapeur, présentant une parfaite étanchéité et ayant une structure optimisée du point de vue de sa fabrication.

Il va de soi que l'invention n'est pas limitée aux formes de réalisation décrites ci-dessus à titre d'exemples mais qu'elle en embrasse au contraire toutes les variantes de réalisation entrant dans le champ de protection défini par les revendications ci-annexées.

## Revendications

1. Dispositif (1) de réglage de débit, à usage médical, comprenant deux pièces (2, 3) reliées l'une à l'autre avec possibilité de déplacement d'une pièce par rapport à l'autre, un organe d'étanchéité (4) placé entre ces deux pièces (2, 3), et des moyens (23, 56) de réglage de débit actionnés par le déplacement d'une pièce (2, 3) par rapport à l'autre, l'une des pièces (2, 3) délimitant un conduit destiné à être raccordé à une source de fluide et l'autre pièce délimitant un conduit destiné à être raccordé à un tuyau d'acheminement dudit fluide ;
dispositif (1) comprenant des moyens de positionnement (15, 35a à 35d, 36a à 36c) permettant de placer les deux pièces (2, 3) dans deux positions différentes, à savoir une première position, dite de "stérilisation", dans laquelle ces deux pièces (2, 3) n'exercent pas, ou exercent peu, de contraintes sur cet organe d'étanchéité (4), et une deuxième position, dite de "fonctionnement", dans laquelle ces deux pièces (2, 3) serrent entre elles ledit organe d'étanchéité (4) de manière à obtenir l'étanchéité requise.

2. Dispositif (1) selon la revendication 1, les moyens de positionnement comprennent :
- au moins une rainure aménagée dans l'une desdites pièces (3) ;
- au moins un bossage (15) solidaire de l'autre pièce (2), coulissant dans cette rainure pour permettre le déplacement des pièces (2, 3) l'une par rapport à l'autre ; et ,
- au moins un dégagement (36a à 36c) communiquant avec la rainure, dans lequel peut être engagé le bossage (15) pour permettre le déplacement de la pièce (3) comprenant la rainure par rapport à la pièce (2) comprenant le bossage (15), de ladite position de fonctionnement à ladite position de stérilisation, et inversement.

3. Dispositif (1) selon la revendication 2, chaque rainure et/ou chaque bossage (15) présentant des zones inclinées (15a) formant des rampes, ces rampes favorisant le passage des pièces (2, 3) de la position de stérilisation à la position de fonctionnement nonobstant la force de rappel élastique de l'organe d'étanchéité (4).

4. Dispositif (1) selon la revendication 2 ou la revendication 3, la rainure présentant au moins une entrée (36a à 36c) dans laquelle un bossage (15) peut être engagé dans une position déterminée d'une pièce (3) par rapport à l'autre, et en ce que l'une des pièces (2) comprend au moins un cran de verrouillage (16) tandis que l'autre pièce (3) comprend au moins un cran de verrouillage (37) correspondant, ces crans (16, 37) étant conformés de telle sorte que le ou les crans (16) d'une pièce (2) peuvent s'encliqueter au-delà du ou des crans (37) de l'autre pièce (3) lors du premier déplacement d'une pièce par rapport à l'autre et être ensuite verrouillés au-delà de ce ou ces derniers de manière à interdire tout retour des pièces (2, 3) vers la position dans laquelle chaque bossage (15) se trouve en regard de chaque entrée (36a à 36c).

5. Dispositif (1) selon l'une des revendications 1 à 4, l'organe d'étanchéité (4) présentant une partie d'étanchéité ayant une section transversale en forme de "U" et en ce que lesdites pièces (2, 3) présentent des formes telles que, lorsqu'elles sont assemblées, elles enserrent cette partie d'étanchéité dans toutes les directions.

6. Dispositif (1) selon l'une des revendications 1 à 5, lesdites pièces (2, 3) étant reliées de manière pivotante l'une par rapport à l'autre, et en ce que l'une desdites pièces (2) comprend une paroi dans laquelle est aménagée, coaxialement à l'axe de pivotement d'une pièce par rapport à l'autre, une rainure circulaire (23) s'étendant sur légèrement moins d'un tour et ayant une profondeur variable sur sa longueur, et le conduit que comprend cette pièce (2) débouche dans cette rainure (23) ; l'autre pièce (3) est solidaire en rotation de l'organe d'étanchéité (4), qui présente un orifice (56) venant en communication d'une part avec le conduit de cette autre pièce et d'autre part avec ladite rainure circulaire (23) ; le pivotement de cette autre pièce (3) par rapport à la première pièce (2) citée permet le déplacement de l'orifice (56) de l'organe d'étanchéité (4) le long de la rainure (23) et donc, du fait de la profondeur différente de cette rainure en différents emplacements de celle-ci, de faire varier la section du conduit d'écoulement du fluide selon la position angulaire d'une pièce (2, 3) par rapport à l'autre.

7. Dispositif (1) selon l'une des revendications 1 à 6, l'une desdites pièces (2, 3) comprenant au moins une fenêtre (14) débouchant sur l'extérieur et en ce que l'autre desdites pièces (3, 2) est solidaire en rotation d'une bague (5) comportant des informations (62) relatives au débit réglé au moyen du dispositif (1), ces informations (62) venant en regard de ladite fenêtre (14) dans les différentes positions angulaires de la pièce (3) solidaire de la bague (5) par rapport à la pièce (2) comportant la fenêtre (14).

## Claims

1. Flowrate control device (1), for medical use, comprising two components (2, 3) connected to one another with possibility of displacement of one component relative to the other, a sealing member (4) placed between these two components (2, 3), and flowrate control means (23, 56) actuated by the displacement of one component (2, 3) relative to the other, one of the components (2, 3) delimiting a conduit intended to be connected to a source of fluid and the other component delimiting a conduit intended to be connected to a tube for delivery of said fluid;
said device (1) comprising positioning means (15, 35a to 35d, 36a to 36c) allowing the two components (2, 3) to be placed in two different positions, namely a first position, called the "sterilizing" position, in which these two components (2, 3) do not exert, or hardly exert, any stresses on this sealing member (4), and a second position, called the "operating" position, in which these two components (2, 3) clamp said sealing member (4) between them in such a way as to obtain the required sealing.

2. Device (1) according to Claim 1, the positioning means comprising;
- at least one groove formed in one of said components (3);
- at least one boss (15) integral with the other component (2) and sliding in this groove in order to permit displacement of the components (2, 3) relative to one another; and
- at least one clearance (36a to 36c) communicating with the groove, in which clearance (36a to 36c) the boss (15) can be engaged in order to permit displacement of the component (3) comprising the groove relative to the component (2) comprising the boss (15), from said operating position to said sterilizing position, and vice versa.

3. Device (1) according to Claim 2, each groove and/or each boss (15) having inclined zones (15a) forming ramps, these ramps facilitating the movement of the components (2, 3) from the sterilizing position to the operating position in spite of the elastic restoring force of the sealing member (4).

4. Device (1) according to Claim 2 or Claim 3, the groove having at least one entry (36a to 36c) in which a boss (15) can be engaged in a defined position of one component (3) relative to the other, and one of the components (2) comprising at least one immobilizing catch (16), while the other component (3) comprises at least one corresponding immobilizing catch (37), these catches (16, 37) being configured in such a way that the catch or catches (16) of one component (2) can snap in beyond the catch or catches (37) of the other component (3) during the first displacement of one component relative to the other and can then be immobilized beyond these in such a way as to prevent any return of the components (2, 3) to the position in which each boss (15) is situated opposite each entry (36a to 36c).

5. Device (1) according to one of Claims 1 to 4, the sealing member (4) having a sealing portion with a U-shaped cross section, and said components (2, 3) having shapes which are such that, when they are joined together, they enclose this sealing portion from all directions.

6. Device (1) according to one of Claims 1 to 5, said components (2, 3) being connected pivotably to one another, and one of said components (2) comprising a wall in which there is formed, coaxially with respect to the axis of pivoting of one component relative to the other, a circular groove (23) which extends along slightly less than one turn and has a variable depth along its length, and the conduit formed in this component (2) opens into this groove (23); the other component (3) being integral in rotation with the sealing member (4) which has an orifice (56) communicating on the one hand with the conduit of this other component and on the other hand with said circular groove (23); the pivoting of this other component (3) relative to the first component (2) mentioned permitting displacement of the orifice (56) of the sealing member (4) along the groove (23) and, consequently, on account of the different depth of this groove at different locations thereof, permitting variation of the cross section of the fluid delivery conduit depending on the angular position of one component (2, 3) relative to the other.

7. Device (1) according to one of Claims 1 to 6, one of said components (2, 3) comprising at least one slot (14) opening to the outside, and the other of said components (3, 2) being integral in rotation with a ring (5) which comprises data (62) relating to Lhe flowrate controlled by means of the device (1), said data (62) coming into line with said slot (14) in the different angular positions of the component (3), integral with the ring (5), relative to the component (2), comprising the slot (14).

## Patentansprüche

1. Vorrichtung (1) zur Durchflussregelung, für den medizinischen Gebrauch, enthaltend zwei Teile (2, 3), die miteinander verbunden sind mit der Möglichkeit, einen der Teile relativ zu dem anderen zu verschieben, ein Abdichtungsorgan (4), das zwischen diesen beiden Teilen (2, 3) angeordnet ist, und Mittel (23, 56) zur Durchfluseregelung, die über die Verschiebung eines der Teile (2, 3) relativ zu dem anderen betätigt werden, wobei eines der Teile (2, 3) eine Rohrleitung begrenzt, die dafür vorgesehen ist, an eine Quelle eines flüssigen oder gasförmigen Mediums angeschlossen zu werden, und das andere Teil eine Rohrleitung begrenzt, die dafür vorgesehen ist, an ein Rohr zur Durchleitung eines flüssigen oder gasförmigen Mediums angeschlossen zu werden;
wobei die Vorrichtung (1) Positioniermittel (15, 35a bis 35d, 36a bis 36c) aufweist, mit denen sich die beiden Teile (2, 3) in unterschiedlichen Positionen anordnen lassen, nämlich in einer ersten Stellung, der sog. "Sterilisationsstellung", in der die beiden Teile (2, 3) das Abdichtungsorgan (4) nicht oder nur geringfügig beanspruchen, und einer zweiten Stellung, der sog. "Arbeitsetellung", in der die beiden Teile (2, 3) das Abdichtungsorgan (4) zwischen sich einklemmen, so dass die gewünschte Abdichtung erreicht wird.

2. Vorrichtung (1) nach Anspruch 1, wobei die Positioniermittel aufweisen:
• zumindest eine Nut, die in einem der Teile (3) ausgebildet ist.
• zumindest einen Höcker (15), der fest mit dem anderen Teil (2) verbunden ist und in dieser Nut gleitet, um das Verschieben der Teile (2, 3) zueinander zu ermöglichen, und
• zumindest eine Aussparung (36a bis 36c), die mit der Nut zusammenwirkt, in die der Höcker (15) eintreten kann, um die Verschiebung des die Nut aufweisenden Teils (3) relativ zu dem den Höcker (15) aufweisenden Teil (2) aus der Arbeitsstellung in die Sterilisationsstellung und umgekehrt zu gestatten.

3. Vorrichtung (1) nach Anspruch 2, wobei jede Nut und/oder jeder Höcker (15) geneigte Bereiche (15a) aufweist, die Rampen bilden, wobei diese Rampen den Übergang der Teile (2, 3) aus der Arbeitsstellung in die Sterilisationsstellung gegen die Rückstellfederkraft des Abdichtungsorgans (4) erleichtern.

4. Vorrichtung nach Anspruch 2 oder Anspruch 3, wobei die Nut zumindest eine Einmündung (36a bis 36c) aufweist, in die ein Höcker (15) in einer festgelegten Stellung des einen Teils (3) relativ zu dem anderen eintreten kann, und das eine der Teile (2) zumindest eine verriegelungseinkerbung (16) aufweist, während das andere Teil (3) zumindest eine korrespondierende Verriegelungseinkerbung (37) aufweist, wobei diese Einkerbungen (16, 37) derart ausgestaltet sind, dass die Einkerbung oder Einkerbungen (16) des einen Teils (2) beim ersten verschieben des einen Teile relativ zu dem anderen Teil jenseits der Einkerbung oder Einkerbungen (37) des anderen Teils (3) einrasten können und anschließend jenseits dieser letzten verriegelt werden können, derart, dass jegliche Rückkehr der Teile (2, 3) in die Stellung verhindert wird, in der jeder Höcker (15) jeder Einmündung (36a bis 36c) zugewandt ist.

5. Vorrichtung (1) nach einem der Ansprüche 1 bis 4, wobei das Abdichtungsorgan (4) einen Abdichtungsbereich mit einem U-förmigen Querschnitt aufweist und die Teile (2, 3) derartige Formen haben, dass sie, wenn sie zusammengefügt wurden, den Abdichtungsbereich in sämtlichen Richtungen einklemmen.

6. Vorrichtung (1) nach einem der Ansprüche 1 bis 5, wobei die Teile (2, 3) drehbeweglich miteinander verbunden sind und eines der Teile (2) eine Wand aufweist, in der in koaxialer Richtung zu der Drehachse des einen Teils relativ zu dem anderen eine kreisförmige Nut (23) ausgebildet ist, die sich leicht über zumindest eine Umdrehung erstreckt und eine in ihrer Länge variable Tiefe hat, und die Rohrleitung, die dieses Teil (2) aufweist, in der Nut (23) mündet; wobei das andere Teil (3) drehfest mit dem Abdichtungsorgan (4) verbunden ist, das eine Öffnung (56) aufweist, die einerseits mit der Rohrleitung des anderen Teils und andererseits mit der kreisförmigen Nut (23) in Zusammenwirkung gebracht wird; wobei die Drehbewegung des anderen Teils (3) relativ zu dem genannten ersten Teil (2) das Verschieben der Öffnung (56) des Abdichtungsorgans (4) entlang der Nut (23) gestattet und aufgrund der an verschiedenen Stellen der Nut unterschiedlichen Tiefe dieser letzten Nut somit bewirkt, dass sich der Querschnitt der Rohrleitung für die Durchleitung des flüssigen oder gasförmigen Mediums entsprechend der winkelstellung des einen Teils (2, 3) relativ zu dem anderen ändert.

7. Vorrichtung (1) nach einem der Ansprüche 1 bis 6, wobei eines der Teile (2, 3) zumindest ein Fenster (14) aufweist, das an der Außenseite mündet und das andere der Teile (3, 2) drehfest mit einem Ring (5) verbunden ist, der Angaben (62) Über den mittels der Vorrichtung (1) regelten Durchfluss enthält, wobei diese Angaben (62) in den verschiedenen Winkelstellungen (3) des mit dem Ring (5) verbundenen Teils (3) relativ zu dem das Fenster (14) aufweisenden Teil (2) dem Fenster (14) zugewandt sind.
